(19) Europäisches Patentamt
European Patent Office
Office européen des brevets

(11) Veröffentlichungsnummer: **0 520 275 A2**

# (12) EUROPÄISCHE PATENTANMELDUNG

(21) Anmeldenummer: **92110041.8**

(51) Int. Cl.[5]: **C07D 277/18**, A01N 43/78

(22) Anmeldetag: **15.06.92**

(30) Priorität: **27.06.91 DE 4121209**

(43) Veröffentlichungstag der Anmeldung:
**30.12.92 Patentblatt 92/53**

(84) Benannte Vertragsstaaten:
**BE CH DE DK ES FR GB GR IT LI NL SE**

(71) Anmelder: **BAYER AG**

**W-5090 Leverkusen 1 Bayerwerk(DE)**

(72) Erfinder: **Baasner, Bernd, Dr.**
**Wagnerstrasse 83**
**W-5060 Bergisch Gladbach 2(DE)**
Erfinder: **Behner, Otto, Dr.**
**In den Birken 83**
**W-5600 Wuppertal 1(DE)**
Erfinder: **Stendel, Wilhelm, Dr.**
**In den Birken 55**
**W-5600 Wuppertal 1(DE)**

## (54) 2-(3-substituierte Phenylhydrazino)-2-thiazoline und 2-(3-substituierte Phenylazo)-2-thiazoline, ihre Herstellung und ihre Verwendung zur Bekämpfung von Ektoparasiten.

(57) Die Erfindung betrifft die Verwendung von 2-(3-substituierte Phenylhydrazino)-2-thiazolinen und 2-(3-substituierte Phenylazo)-2-thiazolinen der allgemeinen Formel (I)

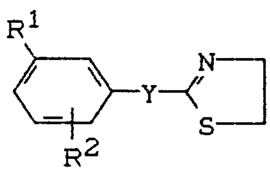

(I)

in der

Y für -NH-NH- oder -N=N- steht und

$R^1$ für Cl oder durch Halogen substituiertes Alkyl steht und

$R^2$ für einen oder mehrere gleiche oder verschiedene Rest aus der Gruppe Wasserstoff, Halogen, Halogenalkyl, Alkoxy, Halogenalkoxy, Alkylthio, Halogenalkylthio, Alkylsulfinyl, Halogenalkylsulfinyl, Alkylsulfonyl, Halogenalkylsulfonyl steht,

zur Bekämpfung von Ektoparasiten sowie neue Thiazoline und ihre Herstellung.

EP 0 520 275 A2

Die Erfindung betrifft teilweise neue 2-(3-substituierte Phenylhydrazino)-2-thiazoline und 2-(3-substituierte Phenylazo)-2-thiazoline, Verfahren zu ihrer Herstellung und ihre Verwendung zur Bekämpfung von Ektoparasiten.

Es ist bereits bekannt, daß im Benzolring substituierte 2-Phenylhydrazino-2-thiazoline und 2-Phenylhydrazo-2-thiazoline ektoparasitäre Wirkungen zeigen (DE-OS 31 33 918; US-P 4 046 753).

Die vorliegende Erfindung betrifft

1. Verwendung von Thiazolinen der allgemeinen Formel (I)

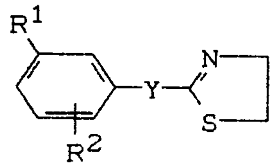

(I)

in der

Y für -NH-NH- oder -N = N- steht und

$R^1$ für Cl oder durch Halogen substituiertes Alkyl steht und

$R^2$ für einen oder mehrere gleiche oder verschiedene Rest aus der Gruppe Wasserstoff, Halogen, Halogenalkyl, Alkoxy, Halogenalkoxy, Alkylthio, Halogenalkylthio, Alkylsulfinyl, Halogenalkylsulfinyl, Alkylsulfonyl, Halogenalkylsulfonyl steht,

zur Bekämpfung von Ektoparasiten.

2. Thiazoline der allgemeinen Formel (I)

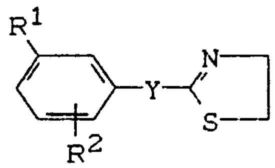

(I)

in der

Y für -NH-NH- oder -N = N- steht und

$R^1$ für Cl oder durch Halogen substituiertes Alkyl steht

$R^2$ für einen oder mehrere gleiche oder verschiedene Rest aus der Gruppe Wasserstoff, Halogen, Halogenalkyl, Alkoxy, Halogenalkoxy, Alkylthio, Halogenalkylthio, Alkylsulfinyl, Halogenalkylsulfinyl, Alkylsulfonyl, Halogenalkylsulfonyl steht,

unter der Voraussetzung, daß $R^1$ und $R^2$ nicht gleichzeitig für Chlor stehen und unter der Voraussetzung wenn $R^1$ $CF_3$ bedeutet, $R^2$ kein zu $CF_3$ paraständiges Chlor oder meta-ständiges $CF_3$ sein darf.

3. Weiterhin wurde ein Verfahren zur Herstellung der Thiazoline der Formel I, in der Y für die Gruppe -NH-NH- steht, gefunden, das dadurch gekennzeichnet ist, daß

a) Phenylhydrazine der Formel (II)

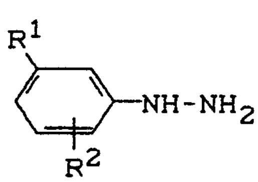

(II)

in der

$R^1$ und $R^2$ die unter 2. angegebene Bedeutung besitzen

mit Thiazolinen der Formel (III)

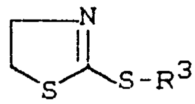 (III)

in der

$R^3$ einen gegebenenfalls substituierten Alkylrest bedeutet,

in Gegenwart von starken Säuren umsetzt oder

b) Thiosemicarbzide der Formel (IV)

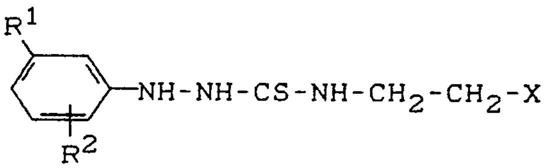 (IV) ,

in der

$R^1$ und $R^2$ die oben angegebene Bedeutung besitzen,

X eine Hydroxylgruppe, eine Alkylsulfonyloxy oder Arylsulfonyloxygruppe oder ein Halogenatom bedeutet,

gegebenenfalls in Gegenwart einer starken Säure cyclisiert oder

c) Isothiosemicarbazide der Formel (V)

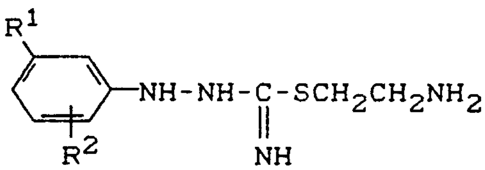 (V)

in der

$R^1$ und $R^2$ die oben angegebene Bedeutung haben,

gegebenenfalls in Gegenwart einer starken Säure cyclisiert oder

d) für den Fall, daS Y für die Azogruppe -N = N-steht,

man Verbindungen der Formel (I), in denen Y für eine Hydrazinogruppe steht und $R^1$ und $R^2$ die oben angegebene Bedeutung haben, mit Hilfe von Oxidationsmitteln dehydriert.

Vorzugweise steht in Formel (I)

$R^1$ für Cl oder 1-5-Halogen-$C_{1-4}$-alkyl,

$R^2$ für einen oder mehrere gleiche oder verschiedene Reste aus der Gruppe Wasserstoff, Chlor, Fluor, Brom, 1-5-Halogen-$C_{1-4}$-alkyl, $C_{1-6}$-Alkoxy, 1-5-Halogen-$C_{1-4}$-alkoxy, $C_1$-$C_6$-Alkylthio, 1-5-Halogen-$C_{1-4}$-alkylthio, $C_{1-6}$-Alkylsulfinyl, 1-5-Halogen-$C_{1-4}$-alkylsulfinyl, $C_{1-6}$-Alkylsulfonyl, 1-5-Halogen-$C_{1-6}$-alkylsulfonyl.

Besonders bevorzugt steht in Formel (I)

$R^1$ für Cl oder 1-5-Halogen-$C_{1-2}$-alkyl,

$R^2$ für einen oder mehrere gleiche oder verschiedene Reste aus der Gruppe Wasserstoff, Chlor, Fluor, Brom, 1-5-Halogen-$C_{1-2}$-alkyl, $C_{1-4}$-Alkoxy, 1-5-Halogen-$C_{1-2}$-alkoxy, $C_{1-2}$-Alkylthio, 1-3-Halogenmethylthio, $C_{1-2}$-Alkylsulfinyl, 1-5-Halogen-$C_{1-2}$-alkylsulfinyl, $C_{1-2}$-Alkylsulfonyl, 1-5-Halogen-$C_{1-2}$-alkylsulfonyl.

Insbesondere steht in Formel (I)

$R^1$ für Cl oder $CF_3$,

$R^2$ für einen oder mehrere Reste aus der Gruppe Wasserstoff, Chlor, Fluor, Brom, $CF_3$, $CHF_2$, $CFCl_2$, $CF_2Cl$, $CCl_3$, $CHCl_2$, $OCH_3$, $OC_2H_5$, $OCF_3$, $OCFCl_2$, $OCF_2Cl$, SMe, SEt, $SCF_3$, $SCFCl_2$, $SF_2Cl$, $CCl_3$, $CH_3SO$, $CF_3SO$, $CH_3SO_2$, $CF_3SO_2$.

Im einzelnen seien folgende Verbindungen der Formel (I) genannt:

2-[(3-Chlorphenyl)hydrazino]-2-thiazolin
2-[(3-Trifluormethyl-phenyl)hydrazino]-2-thiazolin
2-[(3-Chlor-4-trifluormethyl-phenyl)hydrazino]-2-thiazolin
2-[(3-Difluormethylphenyl)hydrazino]-2-thiazolin
2-[(3-Trifluormethyl-6-chlor-phenyl)hydrazino]-2-thiazolin
2-[(3-Fluorphenyl)hydrazino]-2-thiazolin
2-[(3-Bromphenyl)hydrazino]-2-thiazolin
2-[(3-Difluorchlormethyl-phenyl)hydrazino]-2-thiazolin
2-[(3-Fluordichlormethyl-phenyl)hydrazino]-2-thiazolin
2-[(3-Trichlormethyl-phenyl)hydrazino]-2-thiazolin
2-[(3-Methoxyphenyl)hydrazino]-2-thiazolin
2-[(3-Trifluormethoxyphenyl)hydrazino]-2-thiazolin
2-[(3-Methylthiophenyl)hydrazino]-2-thiazolin
2-[(3-Trifluormethylthiophenyl)hydrazino]-2-thiazolin
2-[(3-Trifluormethylsulfinylphenyl)hydrazino]-2-thiazolin
2-[(3-Methylsulfinylphenyl)hydrazino]-2-thiazolin
2-[(3-Methylsulfonylphenyl)hydrazino]-2-thiazolin
2-[(3-Trifluormethylsulfonylphenyl)hydrazino]-2-thiazolin
2-[(3-Chlorphenyl)azo]-2-thiazolin
2-[(3-Trifluormethyl-phenyl)azo]-2-thiazolin
2-[(3-Chlor-4-trifluormethyl-phenyl)azo]-2-thiazolin
2-[(3-Difluormethylphenyl)azo]-2-thiazolin
2-[(3-Trifluormethyl-6-chlor-phenyl)azo]-2-thiazolin
2-[(3-Fluorphenyl)azo]-2-thiazolin
2-[(3-Bromphenyl)azo]-2-thiazolin
2-[(3-Difluorchlormethyl-phenyl)azo]-2-thiazolin
2-[(3-Fluordichlormethyl-phenyl)azo]-2-thiazolin
2-[(3-Trichlormethyl-phenyl)azo]-2-thiazolin
2-[(3-Methoxyphenyl)azo]-2-thiazolin
2-[(3-Trifluormethoxyphenyl)azo]-2-thiazolin
2-[(3-Methylthiophenyl)azo]-2-thiazolin
2-[(3-Trifluormethylthiophenyl)azo]-2-thiazolin
2-[(3-Trifluormethylsulfinylphenyl)azo]-2-thiazolin
2-[(3-Methylsulfinylphenyl)azo]-2-thiazolin
2-[(3-Methylsulfonylphenyl)azo]-2-thiazolin
2-[(3-Trifluormethylsulfonylphenyl)azo]-2-thiazolin.

Neue Verbindungen der Formel (I) werden hergestellt, indem man z.B. nach Verfahren 3a) das 3-Trifluormethylphenylhydrazin mit 2-Methylmercapto-2-thiazolin umsetzt gemäß

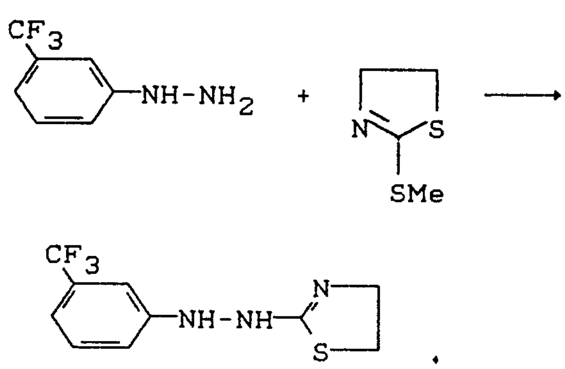

Die Verbindungen der allgemeinen Formel (II) und (III) werden in Gegenwart von Verdünnungsmitteln bei Temperaturen zwischen 20° und 200°C, bevorzugt zwischen 40° und 160°C, am vorteilhaftesten bei der Rückflußtemperatur des eingesetzten Verdünnungsmittels umgesetzt.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäuremethylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid und Alkohole wie Methanol, Ethanol, 2-Propanol, 3-Propanol sowie Mischungen davon, auch mit Wasser.

Die Verbindungen der Formeln (II) und (III) werden in äquimolaren Mengen eingesetzt, ein geringer Überschuß der einen oder anderen Komponente bringt keine wesentlichen Vorteile.

Die Aufarbeitung erfolgt in an sich bekannter Weise, z.B. durch Versetzen der Reaktionsmischung mit Wasser, Abtrennen der organischen Phase, Entfernung des Lösungsmittels und Reinigung des Produktes durch Chromatografie oder Umkristallisation. In Einzelfällen kann das direkt nach Abkühlen des Reaktionsgemisches ausgefallene kristalline Produkt abfiltriert und dann gegebenenfalls gereinigt werden.

Die Verbindungen der Formel II sind bekannt oder können analog zu bekannten Verfahren hergestellt werden /vgl. Houben-Weyl Bd. X/2 (1967) S. 177 ff).

Die Verbindungen der Formel (III) sind bekannt (siehe z.B. Tetrahedron Lett. 1971, 4359).

Die Verbindungen der Formel (I) können weiterhin hergestellt werden, indem man z.B. nach Verfahren 3b das 4-(2-Bromethyl)-1-(3-chlor-4-trifluormethylphenyl)-thiosemicarbazid cyclisiert:

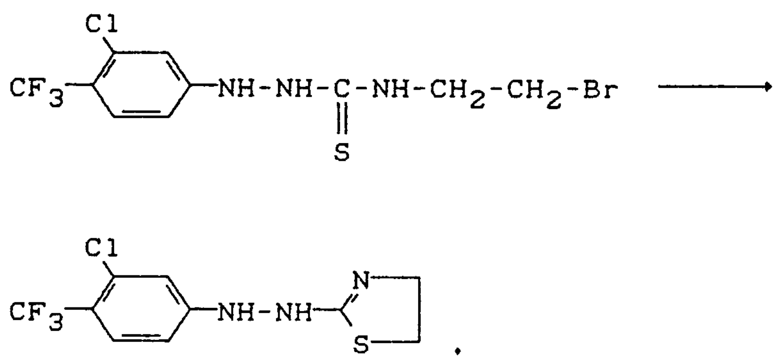

Die Verbindungen der allgemeinen Formel (IV) werden in Gegenwart von Verdünnungsmitteln bei Temperaturen zwischen 20° und 180°C, bevorzugt zwischen 30° und 160°C und am vorteilhaftesten bei der Rückflußtemperatur des eingesetzten Verdünnungsmittels cyclisiert.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäuremethylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid und Alkohole wie Methanol, Ethanol, 2-Propanol, 3-Propanol sowie Mischungen davon, auch mit Wasser.

Die Aufarbeitung erfolgt in an sich bekannter Weise. In der Regel fallen nach Abkühlen des Reaktionsgemisches die Verbindungen kristallin in Form ihrer Salze aus, wobei die Salze gebildet werden mit den Säuren, die während der Reaktion entstehen. Dies sind insbesondere, wenn X für Chlor, Brom oder Arylsulfonyloxy steht, die Chlorwasserstoffsäure, die Bromwasserstoffsäure oder die Arylsulfonsäure. Die Freisetzung erfolgt, indem die Salze in Wasser, gegebenenfalls in der Wärme, gelöst werden und diese Lösung anschließend mit Basen behandelt werden.

Als Basen seien genannt: Alkali-, Erdalkalialkoholate und tertiäre Amine. Besonders bevorzugt seien folgende Basen genannt: Triethylamin, Pyridin, Picoline, Trimethylamin, N-Methylmorpholin, N-Ethylpyrrolidin, Diazabicyclo(4,3,0)-uncecen (DBU), 1,4-Diaza-bicyclo-2,2,2-octan (DABCO), Diazabicyclo(3,2,O)nonen (DBN).

Anschließend wird das in Kälte ausgefallene Produkt abgesaugt, getrocknet und nach bekannten Methoden durch Chromatographie oder durch Umkristallisation gereinigt.

Die Verbindungen der allgemeinen Formel (IV) sind teilweise neu. Man erhält sie auf an sich bekannte Weise durch Umsetzung von Isothiocyanaten der Formel (VI)

$X\text{-}CH_2\text{-}CH_2\text{-}NCS$ (VI),

worin X die oben angegebene Bedeutung hat, mit substituierten Phenylhydrazinen der allgemeinen Formel (II). (vgl. 15 Chem. Lett. 1989, 965 oder Can. J. Chem. 49 (1971), 971

Isothiocyanate der Formel (VI) sind bekannt (siehe z.B. Chem. Lett. 1989, 965).

Phenylhydrazine der Formel (II) sind bekannt oder können nach bekannten Verfahren hergestellt werden (Houben-Weyl Bd. X/2 (1967), 177 ff.).

Weiterhin können die Verbindungen der Formel (I) hergestellt werden, indem man z.B. nach Verfahren 3c gemäß der Formelgleichung

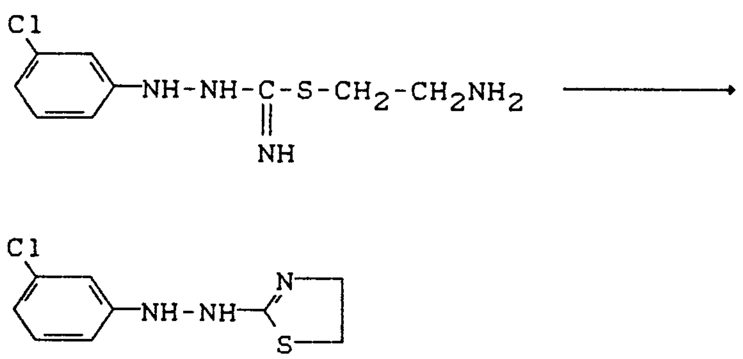

ein Isothiosemicarbazid cyclisiert.

Dazu werden Verbindungen der Formel (V) in Gegenwart von Verdünnungsmitteln zwischen 20° und 200° C, bevorzugt zwischen 40° und 160° C, am vorteilhaftesten bei der Rückflußtemperatur des eingesetzten Lösungsmittels umgesetzt. Die Lösungsmittel werden bei der besonders bevorzugten Ausführungsform aufgeführt.

Die Aufarbeitung erfolgt in an sich bekannter Weise, z.B. durch Versetzen der Reaktionsmischung mit Wasser, Abtrennen der organischen Phase, Entfernung des Lösungsmittels und Reinigung des erhaltenen Rohproduktes durch Chromatografie oder Umkristallisation. Für den Fall, daß das Produkt direkt nach Abkühlen des Reaktionsansatzes ausfällt, wird es abfiltriert und dann gegebenenfalls durch Chromatographie oder Kristallisation gereinigt.

Für den Fall, daß die Verbindungen der Formel (V) in Form eines Salzes, z.B. des Hydrobromides oder Hydrochlorides, eingesetzt werden, fallen die Endprodukte zunächst auch als Hydrobromid oder Hydrochlorid an. In diesen Fällen erfolgt die Freisetzung des Endproduktes nach der unter Verfahren 3b beschriebenen Verfahrensweise.

Die Verbindungen der allgemeinen Formel (V) sind bekannt oder können nach an sich bekannten Verfahren (siehe z.B. Houben-Weyl IX (1955), 912-913) hergestellt werden. Diese Herstellung erfolgt z.B. durch Umsetzung der Thiosemicarbazide der allgemeinen Formel (VII) mit z.B. 2-Bromethylamin oder 2-Chlorethylamin.

Thiosemicarbazide der Formel VII

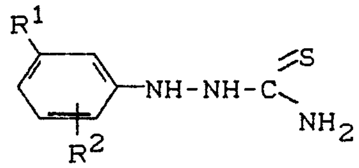

(VII),

in welcher
$R^1$ und $R^2$ die oben angegebene Bedeutung haben,
werden hergestellt durch Umsetzung von Phenylhydrazinen der Formel (II) mit vorzugsweise in situ hergestellter Rhodanwasserstoffsäure (siehe z.B. Indian J. Chem. 233 (1983), 1243). Die Phenylhydrazine (II) werden nach den unter Verfahren 3b zitierten Verfahren hergestellt.

In einer besonderen Ausführungsform wird Verfahren 3c als Einstufenverfahren durchgeführt. So wird z.B. das 3-Chlorphenylthiosemicarbazid mit 2-Bromethylamin zum Thiazolin umgesetzt gemäß:

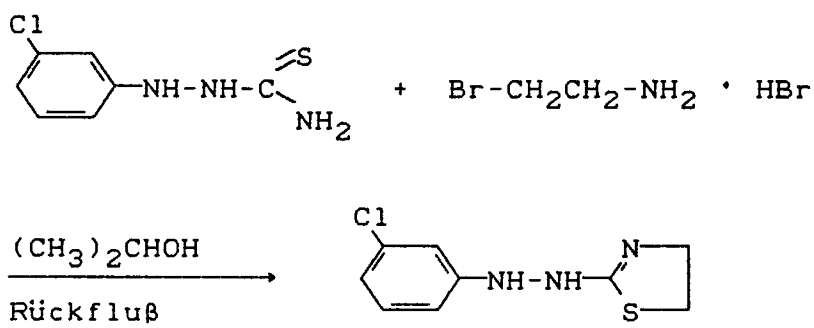


Die Verbindungen der allgemeinen Formel (VII) werden in Gegenwart eines inerten organischen Lösungsmittels bei Temperaturen von 20° bis 220°C, bevorzugt bei 40° bis 200°C, besonders bevorzugt bei der jeweiligen Rückflußtemperatur des eingesetzten Lösungsmittels oder Lösungsmittelgemisches durchgeführt.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäuremethylester und -ethylester, ferner Nitrile, wie z.B. Acetonitril und Propionitril, Benzonitril, Glutarsäuredinitril, darüber hinaus Amine, wie z.B. Dimethylformamid, Dimethylacetamid und N-Methylpyrrolidon, sowie Dimethylsulfoxid, Tetramethylensulfon und Hexamethylphosphorsäuretriamid und Alkohole wie Methanol, Ethanol, 2-Propanol, 3-Propanol, 4-Butanol, 5-Pentanol sowie Mischungen davon, auch mit Wasser.

Die Aufarbeitung erfolgt in an sich bekannter Weise. In der Regel fallen nach Erkalten des Reaktionsgemisches die Verbindungen kristallin an und werden nach Abfiltrieren gereinigt, z.B. durch Chromatografie oder Kristallisation. Werden Bromethylamin oder Chlorethylamin in Form ihrer Bromwasserstoff- oder Chlorwasserstoff-Salze eingesetzt, fallen die Endprodukte auch zunächst in Form der Bromwasserstoff- oder Chlorwasserstoff-Salze an. Zur Freisetzung der Endprodukte werden die Salze in Wasser, gegebenenfalls in der Wärme, gelöst und diese Lösung anschließend mit Basen behandelt.

Als Basen seien genannt: Alkali-, Erdalkalialkoholate und tertiäre Amine. Besonders bevorzugt seien folgende Basen genannt: Triethylamin, Pyridin, Picoline, Trimethylamin, N-Methylmorpholin, N-Ethylpyrrolidin, Diazabicyclo(4,3,0)-undecen (DBU), 1,4-Diaza-bicyclo-2,2,2-octan (DABCO), Diazabicyclo(3,2,0)nonen (DBN).

Anschließend wird das in der Kälte ausgefallene Produkt abfiltriert, getrocknet und nach bekannten Methoden wie z.B. Chromatografie oder Umkristallisation weiter gereinigt.

Die Verbindungen der allgemeinen Formel (VII) und z.B. Bromethylamin oder Chlorethylamin bzw. ihre Hydrogenhalogenide werden in äquimolaren Mengen eingesetzt, ein geringer Überschuß der einen oder

anderen Komponente bringt keine wesentlichen Vorteile.

Verbindungen der allgemeinen Formel (I), in denen Y eine -N = N-(Azo)-gruppierung bedeutet, werden erhalten, indem man z.B. nach Verfahren 3d das 2-[(3-Trifluormethylphenyl)hydrazinol-2-thiazolin mit einem Oxidationsmittel, wie z.B. Wasserstoffperoxid, zum 2-[(3-Trifluormethylphenyl)azol-2-thiazolin umsetzt gemäß Formelschema

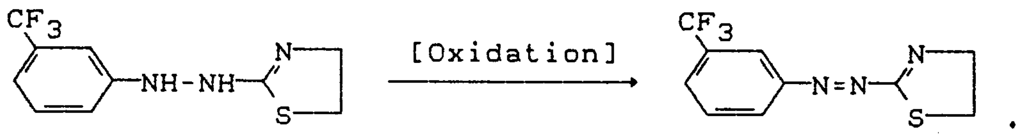


Die Reaktion wird etwa so durchgeführt, daß man Verbindungen der allgemeinen Formel (I) mit Y: -NH-NH-, gegebenenfalls in einem inerten organischen Lösungsmittel, mit einem Oxidationsmittel dehydriert.

Geeignete Oxidationsmittel sind z.B. Metalloxide wie Silberoxid, Bleioxid, Quecksilber, Selenoxid sowie Luftsauerstoff, Eisen(III)chlorid, Bleitetracetat, Quecksilberacetat, Natriumdichromat, Kupfersulfat, Kaliumhexacyanoferrat, salpetrige Säure, verdünnte oder rauchende Salpetersäure, Natriumpersulfat, Natriumhyypochlorid, Permanganate, elementarer Schwefel, Natriumperborat, Wasserstoffperoxid und Chromsäure.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl-, und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäuremethylester und -ethylester, ferner Nitrile, wie z.B. Acetonitril und Propionitril, Benzonitril, Glutarsäuredinitril, weiterhin Alkohole wie Methanol, Ethanol, 2-Propanol, 3-Propanol, Butanol, Pentanol sowie Mischungen davon, auch mit Wasser. Der pH-Wert der Reaktionen kann in weiten Grenzen variiert werden. So können die beispielsweise im sauren Milieu, wie z.B. Eisessig, aber auch in basischem Milieu, wie z.B. wäßriger Natronlauge durchgeführt werden.

Die Reaktionstemperaturen liegen zwischen -30° und +180°C, bevorzugt zwischen 0° und 110°C. Einen Überblick über die Methoden zur Oxidation gibt Houben-Weyl, Bd. X/3 (1965), Seiten 371-380. Die Aufarbeitung erfolgt auf an sich bekannte Weise, z.B. wäßrig/extraktiv oder Abtrennung des Reaktionsproduktes nach Neutralisation durch Extraktion oder Abfiltrieren. Die weitere Reinigung kann beispielsweise durch Chromatographie oder Neutralisation erfolgen.

Die Wirkstoffe eignen sich bei günstiger Warmblütertoxizität zur Bekämpfung von tierischen Schädlingen wie Arthropoden, vorzugsweise Insekten und Spinnentieren, die in der Tierhaltung und Tierzucht bei Haus- und Nutztieren sowie Zoo-, Labor-, Versuchs- und Hobbytieren vorkommen. Sie sind dabei gegen alle oder einzelne Entwicklungsstadien der Schädlinge sowie gegen resistente und normal sensible Arten der Schädlinge wirksam.

Durch die Bekämpfung der tierischen Schädlinge sollen Krankheiten und deren Übertragung, Todesfälle und Leistungsminderungen (z.B. bei der Produktion von Fleisch, Milch, Wolle, Häuten, Eiern) verhindert werden, so daß durch den Einsatz der Wirkstoffe eine wirtschaftlichere und einfachere Tierhaltung möglich ist bzw. in bestimmten Gebieten erst möglich wird.

Zu den Schädlingen gehören:

Aus der Ordnung der Anoplura z.B. Haematopinus spp., Linognathus spp., Solenopotes spp., Pediculus spp., Pthirus spp.;

aus der Ordnung der Mallophaga z.B. Trimenopon spp., Menopon spp., Eomenacanthus spp., Menacanthus spp., Trichodectes spp., Felicola spp., Damalinea spp., Bovicola spp;

aus der Ordnung der Diptera z.B. Chrysops spp., Tabanus spp., Musca spp., Hydrotaea spp., Muscina spp., Haematobosca spp., Haematobia spp., Stomoxys spp., Fannia spp., Glossina spp., Lucilia spp., Calliphora spp., Auchmeromyia spp., Cordylobia spp., Cochliomyia spp., Chrysomyia spp., Sarcophaga spp., Wohlfartia spp., Gasterophilus spp., Oestromyia spp., Oedemagena spp., Hypoderma spp., Oestrus spp., Rhinoestrus spp., Melophagus spp., Hippobosca spp..

Aus der Ordnung der Siphonaptera z.B. Ctenocephalides spp., Echidnophaga spp., Ceratophyllus spp..

Aus der Ordnung der Metastigmata z.B. Hyalomma spp., Rhipicephalus spp., Boophilus spp., Amblyomma spp., Haemaphysalis spp., Dermacentor spp., Ixodes spp., Argas spp., Ornithodorus spp., Otobius spp.;

aus der Ordnung der Mesostigmata z.B. Dermanyssus spp., Ornithonyssus spp., Pneumonyssus spp.

Varroa spec..

Aus der Ordnung der Prostigmata z.B. Cheyletiella spp., Psorergates spp., Myobia spp., Demodex spp., Neotrombicula spp.;

aus der Ordnung der Astigmata z.B. Acarus spp., Myocoptes spp., Psoroptes spp., Chorioptes spp., Otodectes spp., Sarcoptes spp., Notoedres spp., Knemidocoptes spp., Neoknemidocoptes spp. Cytodites spp., Laminosioptes spp..

Zu den Haus- und Nutztieren gehören Säugetiere wie z.B. Rinder, Schafe, Ziegen, Pferde, Schweine, Hunde, Katzen, Kamele, Wasserbüffel, Esel, Kaninchen, Damwild, Rentiere, Pelztiere wie z.B. Nerze, Chinchilla, Waschbär, Vögel wie z.B. Hühner, Puten, Fasane, Gänse, Enten.

Zu Labor- und Versuchstieren gehören z.B. Mäuse, Ratten, Meerschweinchen, Goldhamster, Hunde und Katzen.

Zu den Hobbytieren gehören z.B. Hunde und Katzen.

Die Anwendung kann sowohl prophylaktisch als auch therapeutisch erfolgen.

Die Anwendung der Wirkstoffe erfolgt direkt oder in Form von geeigneten Zubereitungen enteral, parenteral, dermal, nasal, durch Behandlung der Umgebung oder mit Hilfe wirkstoffhaltiger Formkörper wie z.B. Streifen, Platten, Bänder, Halsbänder, Ohrmarken, Gliedmaßenbänder, Markjerungsvorrichtungen.

Die enterale Anwendung der Wirkstoffe geschieht z.B. oral in Form von Pulver, Tabletten, Kapseln, Pasten, Boli, Tränken, Granulaten, oral applizierbaren Lösungen, Suspensionen oder Emulsionen, medikiertem Futter oder Trinkwasser. Die dermale Anwendung geschieht z.B. in Form des Tauchens (Dippen), Sprühens (Sprayen) oder Aufgießens (pour-on und spot-on) und des Einpuderns. Die parenterale Anwendung geschieht z.B. in Form der Injektion (z.B. intramusculär, subcutan, intravenös) oder durch Implantate.

Besonders hervorgehoben seien die Zubereitungen zur dermalen Anwendung. Dazu gehören Lösungen, Suspensions- und Emulsionskonzentrate sowie Mikroemulsionen, die vor Anwendung mit Wasser verdünnt werden, Aufgußformulierungen, Pulver und Stäube, Aerosole und wirkstoffhaltige Formkörper sowie dust-bags, back-rubber.

Zu den oberflächenaktiven Stoffen zählen:

Emulgatoren und Netzmittel wie anionaktive Tenside, z.B. Alkylsulfonate, Alkylsulfate, Arylsulfonate, Na-Laurylsulfate, Fettalkoholethersulfate, Mono/Dialkylpolyglykoletherorthophosphorsäureester-Monoethanolaminsalz, Calciumalkylarylsulfonat;

kationaktive Tenside, z.B. Cetyltrimethylammoniumchlorid;

ampholytische Tenside, z.B. Di-Na-N-lauryl-betaiminodipropionat oder Lecithin;

nichtionogene Tenside, z.B. polyoxyethyliertes Ricinusöl, polyoxyethyliertes Sorbitan-Monooleat, polyoxyethyliertes Sorbitan-Monostearat, Glycerinmonostearat, Polyoxyethylenstearat, Alkylphenolpolyglykolether, polyoxyethyliertes Sorbitanmonopalmitat, Polyoxyethylenlaurylether, Polyoxyethylen-oleylether, Polyoxyethylenmannitanmonolaurat, Alkylpolyglykolether, Oleylpolyglykolether, Dodecylpolyglykolether, ethoxyliertes Nonylphenol, Isooctylphenolpolyethoxyethanol.

Die Zubereitungen können außerdem enthalten:

Haftvermittler, z.B. Carboxymethylcellulose, Methylcellulose und andere Cellulose- und Stärke-Derivate, Polyacrylate, Alginate, Gelatine, Gummi arabicum, Polyvinylpyrrolidon, Polyvinylalkohol, Copolymere aus Methylvinylether und Maleinsäureanhydrid, Polyethylenglykole, Paraffine, Öle, Wachse, hydriertes Rizinusöl, Lecithine und synthetische Phospholipide.

Die Zubereitungen können Farbstoffe wie anorganische Pigmente, z.B.Eisenoxid, Titanoxid, Ferrocyanblau und organische Farbstoffe, wie Alizarin-, Azo- und Metallphthalocyaninfarbstoffe enthalten.

Die Zubereitungen können Spreitmittel enthalten, z.B. Silikonöle verschiedener Viskosität, Fettsäureester wie Ethylstearat, Di-n-butyl-adipat, Laurinsäurehexylester, Dipropylen-glykolpelargonat, Ester einer verzweigten Fettsäure mittlerer Kettenlänge mit gesättigten Fettalkoholen $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester, Dibutylphthalat, Adipinsäurediisopropylester, letzterem verwandte Estergemische u.a.;

Triglyceride wie Capryl/Caprinsäuretriglycerid, Triglyceridgemische mit Pflanzenfettsäuren der Kettenlänge $C_8$-$C_{12}$ oder anderen speziell ausgewählten natürlichen Fettsäuren, Partialglyceridgemische gesättigter oder ungesättigter evtl. auch hydroxylgruppenhaltige Fettsäuren, Monodiglyceride der $C_8$/$C_{10}$-Fettsäuren und andere; Fettalkohole wie Isotridecylalkohol, 2-Octyldodecanol, Cetylstearylalkohol, Oleylalkohol.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind gegebenenfalls gebrochene und fraktionierte, z.B. synthetische und natürliche Gesteinsmehle wie Kaoline, Talkum, Kreide, Quarz, Diatomeenerde,

Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken, Sägemehl.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in Form ihrer oben erwähnten festen oder flüssigen Formulierungen auch eingekapselt vorliegen.

Die Wirkstoffe können auch in Form eines Aerosols angewendet werden. Dazu wird der Wirkstoff in geeigneter Formulierung unter Druck fein verteilt.

Es kann auch vorteilhaft sein, die Wirkstoffe in Formulierungen anzuwenden, die den Wirkstoff verzögert freigeben. Als solche seien wirkstoffhaltige Formkörper wie z.B. Platten, Bänder, Streifen, Halsbänder, Ohrmarken, Schwanzmarken, Gliedmaßenbänder, Halfter, Markierungsvorrichtungen genannt. Als solche seien auch wirkstoffhaltige Implantate und Boli genannt.

Die Verabreichung der Wirkstoffe kann auch zusammen mit dem Futter und/oder dem Trinkwasser erfolgen.

Die Wirkstoffe können in den Formulierungen allein oder in Mischung mit anderen Wirkstoffen oder Synergisten vorliegen.

Direkt angewendete Formulierungen enthalten zwischen $10^{-7}$ und 5 Gewichtsprozent, bevorzugt zwischen $10^{-4}$ und 1 Gewichtsprozent Wirkstoff.

Formulierungen die erst nach weiterer Verdünnung angewendet werden enthalten 1 - 95 Gewichtsprozent, bevorzugt 5 - 90 Gewichtsprozent Wirkstoff.

Beispiel A

Zecken In vivo-Minidiptest

Testtiere:

Boophilus microplus (alle parasitierenden Entwicklungsstadien/Larven, Metalarven, Nymphen, Metanymphen, Adulte) von Boophilus microplus (SP-resistenter Parkhurst-Stamm).

Lösungsmittel:

35 Gewichtsteile Ethylenglykolmonomethylether
35 Gewichtsteile Nonylphenolpolyglykolether

Wirtstiere:

Rinder werden vor Versuchsbeginn in der Weise geschoren, daß auf der Rücken- und Seitenpartie behaarte Felder von der Größe 8 x 8 cm stehen gelassen werden. Der Abstand der Felder beträgt ca. 20 cm und sie sind diagonal versetzt angeordnet.

Testverfahren:

Rinder werden 12 x in 2tägigem Abstand mit je 2000-4000 nüchternen 14-28 Tage alten Larven von Boophilus microplus infiziert. Zählung der vom 21. - 23. Tag p.i. zur Entwicklung kommenden weiblichen Adulten als Maß für die Infektionsstärke auf den einzelnen Feldern. Am 24. Tage intensives Benetzen (2 Minuten) der einzelnen Felder mit 200 ml einer Verdünnung der zu testenden formulierten Substanz durch Aufsetzen des Mini-Dip-Gerätes. Zwecks Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die jeweils gewünschte Konzentration. Nach Abtrocknen der behandelten Felder werden Gazebeutel mit Hilfe eines hautfreundlichen Klebstoffs so um die Felder befestigt, daß sie diese vollkommen einschließen. Die Beutel sind aus Gaze gefertigt und haben

eine zylindrische Form, ⌀ 12 cm, mit einem angesetzten 3 cm breiten Fußring. Dieser Beutel wird durch Gummiringe verschlossen.

Testkriterien:

Vom 24. - 45. Tag p.i. Zählung der auf dem Tier in den einzelnen Beuteln zur Entwicklung kommenden weiblichen Adulten. Als Kriterien für die Wirkung gilt die Verminderung der Zahl der zur Entwicklung kommenden weiblichen Adulten mit fertilen Gelegen auf den behandelten Feldern im Vergleich zu unbehandelten Kontrollfeldern. Die Wirksamkeit drückt man in % aus. Dabei bedeutet, daß alle Zecken abgetötet wurden, 0 % bedeutet, daß keine Zecken abgetötet wurden,

| Wirkstoff Bsp. Nr. | Wirkstoffkonz. ppm a.i. | Abtötende Wirkung in % Boophilus microplus im Mini-Dip |
|---|---|---|
| 1 | 300<br>100<br>30 | 100<br>100<br>100 |

Beispiel B

Räudemilbentest

Testtiere: Psoroptes ovis (Larven, Nymphen, Adulte)
Lösungsmittel: 35 Gewichtsteile Ethylenglykolmonomethylether
35 Gewichtsteile Nonylphenopolyglykolether

Zwecks Herstellung einer geeigneten Formulierung vermischt man drei Gewichtsteile Wirkstoff mit sieben Teilen des oben angegebenen Lösungsmittel-EmulgatorGemisches und verdünnt das so erhaltene Emulsionskonzentrat mit Wasser auf die jeweils gewünschte Konzentration.

1 ml dieser Wirkstoffzubereitung wird in PP-Blisterfolien entsprechender Größe pipettiert. Anschließend werden ca. 25 Milben in die Wirkstoffzubereitung überführt.

Nach 24 Stunden wird die Wirksamkeit der Wirkstoffzubereitung bestimmt. Die Wirksamkeit drückt man in % aus. Dabei bedeutet 100 %, daß alle Milben abgetötet wurden; 0 % bedeutet, daß keine Milben abgetötet wurden.

| Wirkstoff Bsp. Nr. | Wirkstoffkonz. ppm a.i. | Abtötende Wirkung in % Psoroptes ovis |
|---|---|---|
| 6 | 10<br>1 | 100<br>100 |

Beispiele

a) Allgemeine Vorschrift zur Herstellung der Verbindungen der allgemeinen Formel (I) (Y = NH-NH) gemäß Verfahren 3c) in seiner speziellen Ausführungsform

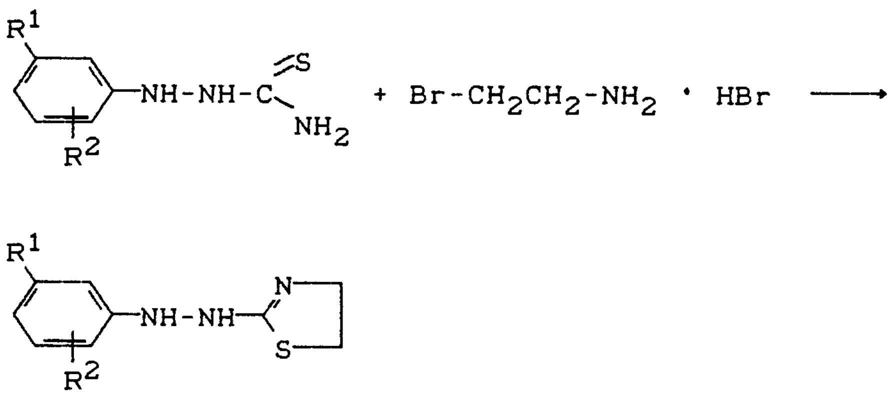

0,15 Mol 1-(3-subst. Phenyl)-3-thiosemicarbazid und 0,15 Mol 2-Bromethylamin-hydrobromid werden in 800 ml Isopropanol 12 h zum Rückfluß erhitzt. Nach dem Abkühlen wird mit gesättigter Natriumhydrogencarbonat-Lösung verrührt, mit Methylenchlorid ausgeschüttelt und die flüchtigen Bestandteile im Vakuum abgezogen. Der Rückstand wird aus Petrolether umkristallisiert. Gemäß diesem Verfahren werden die in der folgenden Tabelle 1 aufgeführten Verbindungen der allgemeinen Formel (I) mit Y = -NH-NH- erhalten.

Tabelle 1

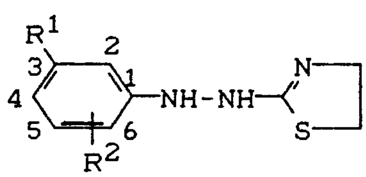

(I)

| Bsp. Nr. | $R^1$ | $R^2$ | Fp. [°C] |
|---|---|---|---|
| 1 | Cl | H | 132-133 |
| 2 | $CF_3$ | H | 117-118 |
| 3 | Cl | 4-$CF_3$ | 155 |
| 4 | $CHF_2$ | H | |
| 5 | $CF_3$ | 6-Cl | |

b) Allgemeine Vorschrift zur Herstellung der Verbindungen der allgemeinen Formel (I) mit Y : -N=N- durch Oxidation

α) mit $H_2O_2$

Ein Gemisch aus 0,05 Mol 2-(3-substituiertes Phenylhydrazino)-2-thiazolin in 120 ml Methylenchlorid und 100 ml 2 normaler Natronlauge wird mit 7 ml 30 %igem $H_2O_2$ versetzt und 18 h zum Rückfluß erhitzt. Nach 8 h und dann weiteren 6 Stunden werden jeweils 2 ml 30 %iges $H_2O_2$ nachgegeben. Anschließend wird die organische Phase abgetrennt, die wäßrige Phase mit Methylenchlorid extrahiert, die vereinigten organischen Phasen im Vakuum eingeengt und der Rückstand aus Hexan/Ether umkristallisiert.

β) mit Silberoxid

Zu einer Lösung von 0,02 Mol 2-(3-substituiertes Phenyl)-3-thiosemicarbazid in 200 ml Essigester werden 1,7 g Silberoxid gegeben. Anschließend wird 18 h lang bei Raumtemperatur gerührt, dann abfiltriert, im Vakuum eingeengt und der kristalline Rückstand mit Petrolether verrührt.

Gemäß diesen Verfahren α) und/oder β) werden die in der folgenden Tabelle 2 aufgeführten Verbindungen der allgemeinen Formel (I) mit Y : -N = N- erhalten.

Tabelle 2

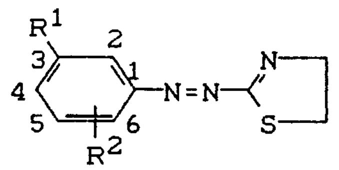

(I)

| Bsp. Nr. | $R^1$ | $R^2$ | Fp. [°C] |
|---|---|---|---|
| 6 | Cl | H | 92 |
| 7 | $CF_3$ | H | 68 |
| 8 | Cl | 4-$CF_3$ | 83 |
| 9 | $CHF_2$ | H | |
| 10 | $CF_3$ | 6-Cl | |

## Patentansprüche

**1.** Verwendung von Thiazolinen der allgemeinen Formel (I)

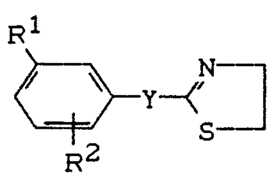

(I)

in der

Y für -NH-NH- oder -N = N- steht und

$R^1$ für Cl oder durch Halogen substituiertes Alkyl steht und

$R^2$ für einen oder mehrere gleiche oder verschiedene Rest aus der Gruppe Wasserstoff, Halogen, Halogenalkyl, Alkoxy, Halogenalkoxy, Alkylthio, Halogenalkylthio, Alkylsulfinyl, Halogenalkylsulfinyl, Alkylsulfonyl, Halogenalkylsulfonyl steht,

zur Bekämpfung von Ektoparasiten.

**2.** Thiazoline der allgemeinen Formel (I)

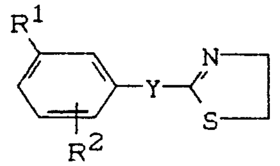

(I)

in der
Y für -NH-NH- oder -N = N- steht und
$R^1$ für Cl oder durch Halogen substituiertes Alkyl steht
$R^2$ für einen oder mehrere gleiche oder verschiedene Rest aus der Gruppe Wasserstoff, Halogen, Halogenalkyl, Alkoxy, Halogenalkoxy, Alkylthio, Halogenalkylthio, Alkylsulfinyl, Halogenalkylsulfinyl, Alkylsulfonyl, Halogenalkylsulfonyl steht,

unter der Voraussetzung, daß $R^1$ und $R^2$ nicht gleichzeitig für Chlor stehen und unter der Voraussetzung wann $R^1$ $CF_3$ bedeutet, $R^2$ kein zur $CF_3$-Gruppe para- ständiges Chlor oder meta-ständiges $CF_3$ sein darf.

**3.** Verfahren zur Herstellung der Thiazoline der Formel (I),

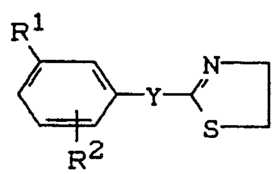

(I)

in der
Y für -NH-NH- oder -N = N- steht und
$R^1$ für Cl oder durch Halogen substituiertes Alkyl steht
$R^2$ für einen oder mehrere gleiche oder verschiedene Rest aus der Gruppe Wasserstoff, Halogen, Halogenalkyl, Alkoxy, Halogenalkoxy, Alkylthio, Halogenalkylthio, Alkylsulfinyl, Halogenalkylsulfinyl, Alkylsulfonyl, Halogenalkylsulfonyl steht,

unter der Voraussetzung, daß $R^1$ und $R^2$ nicht gleichzeitig für Chlor stehen und unter der Voraussetzung wenn $R^1$ $CF_3$ bedeutet, $R^2$ kein zur $CF_3$-Gruppe para- ständiges Chlor oder meta-ständiges $CF_3$ sein darf.

das dadurch gekennzeichnet ist, daß
a) Phenylhydrazine der Formel (II)

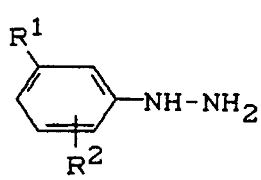

(II)

in der
$R^1$ und $R^2$ die oben angegebene Bedeutung besitzen
mit Thiazolinen der Formel (III)

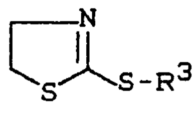

(III)

in der

$R^3$ einen gegebenenfalls substituierten Alkylrest bedeutet,

in Gegenwart von starken Säuren umsetzt oder

b) Thiosemicarbzide der Formel (IV)

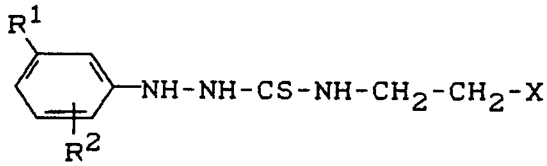

(IV) ,

in der

$R^1$ und $R^2$ die oben angegebene Bedeutung besitzen,

X eine Hydroxylgruppe, eine Alkylsulfonyloxy- oder Arylsulfonyloxygruppe oder ein Halogenatom bedeutet,

gegebenenfalls in Gegenwart einer starken Säure cyclisiert oder

c) Isothiosemicarbazide der Formel (V)

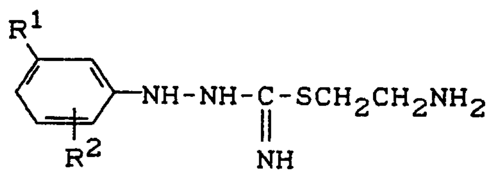

(V)

in der

$R^1$ und $R^2$ die oben angegebene Bedeutung haben,

gegebenenfalls in Gegenwart einer starken Säure cyclisiert, oder

d) für den Fall, daß Y für die Azogruppe -N = N-steht

man Verbindungen der Formel (I), in denen Y für eine Hydrazinogruppe steht und $R^1$ und $R^2$ die oben angegebene Bedeutung haben, mit Hilfe von Oxidationsmitteln dehydriert.

**4.** Ektoparasitizide Mittel, gekennzeichnet durch einen Gehalt an mindestens einem Thiazolin der Formel (I) gemäß Anspruch 1.

**5.** Verfahren zur Bekämpfung von Ektoparasiten, dadurch gekennzeichnet, daß man Thiazoline der Formel (I) gemäß Anspruch 1 auf Ektoparasiten und/oder ihren Lebensraum einwirken läßt.

**6.** Verfahren zur Herstellung von ektoparasitiziden Mitteln, dadurch gekennzeichnet, daß man Thiazoline der Formel (I) gemäß Anspruch 1 mit Streckmitteln und/oder oberflächenaktiven Mitteln vermischt.

**7.** Verwendung von Thiazolinen der Formel (I) gemäß Anspruch 1 zur Herstellung von ektoparasitiziden Mitteln.